# FASCICULE DE BREVET EUROPEEN

(11) **EP 0 732 918 B1**
(45) Date de publication et mention de la délivrance du brevet: **28.06.2000**
(21) Numéro de dépôt: 95907688.6
(22) Date de dépôt: 23.01.1995
(51) Int. Cl.: A61K 31/14, A61K 33/16, A61K 47/18

(54) **SOLUTION INJECTABLE DE FLUORURE DE BENZALKONIUM**
INJIZIERBARE LOESUNG ENTHALTEND BENZALKONIUMFLUORID
BENZALKONIUM FLUORIDE INJECTABLE SOLUTION

(30) Priorité: 24.01.1994 FR 9400695
(43) Date de publication de la demande: 25.09.1996
(73) Titulaire: Aromafarm Ltd., Castletown, Isle of Man (GB)
(72) Inventeur: LAGNY, Pierre, Kildare (IE); BOURBON, Pierre, F-31000 Toulouse (FR)
(74) Mandataire: Keib, Gérard
(86) Numéro de dépôt international: FR9500070
(87) Numéro de publication internationale: WO9519766

(56) Documents cités:
- EP-A- 0 046 594
- EP-A- 0 308 564

## Description

L'invention concerne une solution administrable par voie intravasculaire comprenant au moins un fluorure de benzalkonium.

EP-A-0.308.564 mentionne déjà l'utilisation du fluorure de benzalkonium pour obtenir une composition administrable par voie générale ou parentérale comprenant entre 0,05 % et 7 %, notamment de l'ordre de 1 % en poids de fluorure de benzalkonium, destinée à lutter contre les virus ou les rétrovirus, notamment l'herpès ou les LVA responsables du SIDA.

Néanmoins, le fluorure de benzalkonium est supposé, comme tous les sels de benzalkonium qui sont tensio-actifs, détenir un fort pouvoir hémolytique prohibant son administration par voie intravasculaire. Ainsi, bien que l'administration générale ou parentérale du fluorure de benzalkonium soit citée dans EP-A-0.308.564, son utilisation en pratique se heurtait à cette difficulté considérée jusqu'à maintenant comme insurmontable pour deux raisons : la concentration à atteindre dans le sang pour procurer une activité était elle-même supposée engendrer l'hémolyse du sang ; l'injection, même pratiquée très progressivement, suppose la création localement d'une concentration élevée, donc hémolytique, du fluorure de benzalkonium. En particulier, l'injection de ce sel à des concentrations de l'ordre de 1 % en poids s'est avérée impossible. Or, cette concentration était considérée comme indispensable pour obtenir une activité suffisante. On avait donc renoncé à employer les sels de benzalkonium, et plus particulièrement le fluorure de benzalkonium, par voie intravasculaire, en considérant que la dimunution des concentrations des solutions injectées aurait supprimé toute activité virucide ou bactéricide compte tenu des CMI (concentrations minimales inhibitrices) mesurées in vitro, et que, même à ces faibles concentrations, un très grand risque d'hémolyse, et donc de décès immédiat, persiste. Dès lors, l'administration intravasculaire à l'homme ou à l'animal des sels de benzalkonium, et notamment du fluorure de benzalkonium, était jusqu'alors purement et simplement proscrite.

Or, un besoin constant se fait sentir de disposer d'un traitement de choc contre les affections virales ou infectieuses, notamment d'origine immunodéficitaire, et plus particulièrement d'un traitement des malades atteints du SIDA en phase déclarée ou évolutive qui présentent des infections opportunistes sévères aboutissant rapidement à une déshydratation et à la mort.

Un tel traitement de choc pose néanmoins une série de problèmes apparemment contradictoires :
- il doit agir, non seulement contre les micro-organismes pathogènes responsables de l'état clinique détérioré du patient, mais également contre les virus responsables de l'immunodéficience (cas du SIDA),
- il doit disposer d'un spectre d'action très large compte tenu de la grande variété des affections qui peuvent être en cause dans le cas d'une immunodéficience,
- il doit cependant être exempt d'effets secondaires, et en particulier ne pas irriter ou attaquer les organes ou les cellules saines de l'organisme déjà considérablement affaibli.

L'invention vise donc, d'une façon générale, à procurer une solution à ce problème général qui, jusqu'à maintenant, n'est pas résolu. Elle vise donc à proposer un traitement de malades atteints d'affections virales ou infectieuses sévères, notamment multiples, telles que celles dues à une immunodéficience.

Plus particulièrement, les inventeurs ont constaté avec surprise que, parmi les ammoniums quaternaires, le fluorure de benzalkonium pouvait être effectivement injecté par voie intravasculaire sans risque d'hémolyse dans une fourchette de concentrations précise avec laquelle une activité effective a été cependant constatée.

L'invention vise donc plus particulièrement à proposer une solution injectable d'un agent antiviral ou anti-infectieux tel qu'un sel de benzalkonium.

Pour ce faire, l'invention concerne une solution administrable par voie intravasculaire caractérisée en ce qu'elle comprend de l'ordre de ou moins de 0,23 % en poids d'au moins un fluorure de benzalkonium de formule : 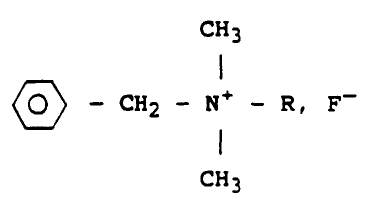 ou R est un radical alcoyle pouvant varier entre C₈H₁₇ et C₁₈H₃₇ , dissous dans un excipient injectable par voie intravasculaire.

On a pu en effet constater que lorsque les proportions en poids de fluorure de benzalkonium sont de l'ordre de ou inférieures à 0,23 %, et notamment de l'ordre de 0,20 % en poids, aucune hémolyse n'est à craindre en général, contrairement au préjugé général de l'art antérieur. De plus, une amélioration clinique peut être constatée en utilisant des concentrations supérieures à 0,05 % en poids de fluorure de benzalkonium. Ainsi, la solution selon l'invention comprend de 0,05 à 2,3 g/l - notamment de l'ordre de 2,1 g/l- de fluorure de benzalkonium, de préférence un fluorure de benzalkonium dans lequel R est C₁₄H₂₉.

Plus particulièrement, la solution selon l'invention comprend une concentration de fluorure de benzalkonium déterminée pour pouvoir administrer en injection lente des doses unitaires de l'ordre de 0,5 à 23 mg -notamment de l'ordre de 21 mg- de fluorure de benzalkonium par litre de sang du patient à traiter. Ainsi, dans le cas d'un homme ayant cinq litres de sang, on réalise une dose injectée de 2,5 à 130 mg, notamment de l'ordre de 105 mg, de fluorure de benzalkonium.

Ainsi, selon l'invention, deux conditions doivent être réunies pour permettre l'injection intravasculaire du fluorure de benzalkonium : la concentration en fluorure de benzalkonium dans la solution injectée doit être inférieure à 0,23 % ; et la quantité totale injectée par litre de sang du patient doit rester inférieure à 23 mg.

Pour déterminer la concentration du fluorure de benzalkonium dans la solution à injecter, on prélève du sang du patient immédiatement avant l'injection, on soumet ce sang à des essais hémolytiques in vitro avec des solutions comprenant initialement 0,23 % de fluorure de benzalkonium, et on diminue les concentrations jusqu'à obtenir une solution non hémolytique.

Selon l'invention, on vérifie préalablement à l'injection le caractère non hémolytique de la solution en déterminant la concentration hémolytique sur le sang du patient.

La solution selon l'invention peut être conditionnée en ampoules de 5 à 15 ml -notamment de 10 ml-comprenant entre 0,5 et 23 mg -notamment 21 mg pour une ampoule de 10 ml- de fluorure de benzalkonium en respectant la valeur maximum de 0,23 %. Et on injecte entre 5 et 15 ml, notamment 10 ml, de solution par litre de sang du patient, notamment 50 ml dans le cas de l'homme.

La solution selon l'invention est avantageusement constituée de fluorure de benzalkonium dissous dans un soluté physiologique. R est avantageusement compris entre C₁₂H₂₅ et C₁₄H₂₉. Selon l'invention, la solution peut comporter, en outre, au moins un dérivé métallique du fluor, et plus particulièrement du fluorure de lithium.

L'invention concerne en outre l'application d'au moins un fluorure de benzalkonium dont la formule est mentionnée ci-dessus, dissous à une concentration de l'ordre de ou inférieure à 0,23 % en poids dans un excipient injectable par voie intravasculaire pour obtenir une solution administrable par voie intravasculaire destinée à lutter contre les maladies virales ou infectieuses, notamment d'origine immunodéficitaire. Plus particulièrement, l'invention concerne l'application d'au moins un tel fluorure de benzalkonium dissous à une concentration de l'ordre de ou inférieure à 0,23 % en poids dans un excipient injectable par voie intravasculaire pour obtenir une solution administrable par voie intravasculaire destinée à lutter contre le SIDA en phase déclarée ou évolutive. L'invention concerne donc une méthode de traitement des maladies virales ou infectieuses, notamment d'origine immunodéficitaire, telles que le SIDA en phase déclarée ou évolutive, dans laquelle on injecte par voie intravasculaire une solution selon l'invention. Dans la méthode utilisée, on injecte une solution après avoir déterminé ex vivo le pouvoir hémolytique du fluorure de benzalkonium vis-à-vis du sang du patient, la solution injectée ayant une concentration inférieure à cette concentration hémolytique, et on administre en injection lente une quantité de fluorure de benzalkonium comprise entre 0,5 et 23 mg par litre de sang du patient à traiter, notamment et de préférence entre 20 et 23 mg.

Ainsi, dans une application selon l'invention, on utilise entre 0,5 et 23 mg -notamment de l'ordre de 21 mg- de fluorure de benzalkonium dissous dans 10 ml de soluté.

L'invention est donc particulièrement utile pour traiter des maladies virales qui détruisent les défenses immunitaires, et plus particulièrement à titre de traitement de choc. L'invention a ainsi permis de traiter effectivement des cas de maladies de Carré et des cas de SIDA en phase avancée, c'est-à-dire développant des maladies infectieuses opportunistes.

D'autres caractéristiques et avantages de l'invention apparaîtront des exemples donnés ci-après.

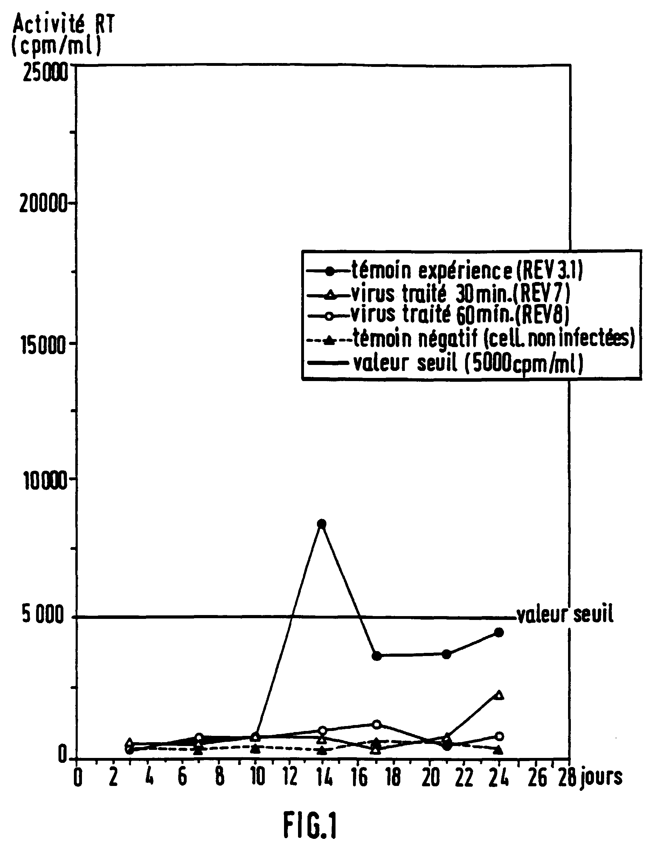

Les figures 1 à 4 annexées représentent des courbes de résultats d'essais de mesure in vitro de l'activité virucide sur le virus VIH-1 du fluorure de benzalkonium conformément à l'exemple 2 décrit ci-après.

### ESSAIS VIRUCIDES IN VITRO

### Exemple 1

On a déterminé l'activité virucide du fluorure de tétradécyl-diméthyl-benzyl-ammonium (FBK) vis-à-vis des virus de vertébrés conformément à la norme française AFNOR T 72-180, décembre 1989. Ce fluorure de benzalkonium a été dilué en solution dans l'eau distillée stérile de concentrations suivantes : 0,02 ; 0,01 ; 0,005 % (masse divisée par volume).

Les essais ont été réalisés sur les souches de virus suivantes :
- picornaviridae : enterovirus polio 1, souche SABIN, cultivé sur cellule VERO,
- adenoviridae : adenovirus type 5, cultivé sur cellule KB,
- herpetoviridae : Herpès Simplex Virus Type 1 (HSV 1) et Herpès Simplex Virus Type 2 (HSV 2) fournis par l'Institut Pasteur et cultivés sur cellule MCR-5.

L'eau, les milieux de culture et les réactifs sont ceux préconisés par la norme AFNOR T72-180. Le titrage des virus se fait par la lecture de l'essai cytopathique, suivant la méthode décrite dans la norme AFNOR T72-180. Le calcul du titre infectieux est effectué par la méthode de FISHER et YATES à l'aide des tables de WYSHAK et DETRE. Les résultats sont donnés en nombre d'unités infectieuses par ml, ou d'unités formant plage par ml (UFP/ml).

On a tout d'abord recherché le seuil de toxicité (DL0) du fluorure de benzalkonium. Avant filtration, ce seuil de toxicité était de 0,05 % (en mg par ml). Après filtration, le seuil de toxicité était de 0,01 %.

La capacité des cellules traitées par le fluorure de benzalkonium à développer l'infection virale a été déterminée et les résultats sont fournis dans le tableau 1.

**Tableau 1**

| CAPACITE DES CELLULES TRAITEES PAR LE FBK A DEVELOPPER L'INFECTION VIRALE Titre des suspensions virales (UFP/ml) | | |
|---|---|---|
| Souches | Contrôle | Après contact avec le fluorure de benzalkonium |
| ENTEROVIRUS POLIO 1 | 10^{7,68} | 10^{6,98} |
| ADENOVIRUS Type 5 | 10^{8,64} | 10^{7,56} |
| HERPES SIMPLEX VIRUS Type 1 (HSV 1) | 10^{8,00} | 10^{7,02} |
| HERPES SIMPLEX VIRUS Type 2 (HSV 2) | 10^{8,50} | 10^{7,84} |

L'activité virucide a ensuite été déterminée par la technique dite de tamisage moléculaire comprenant une filtration sur gel de séphadex LH 20 et l'utilisation de concentrations subcytotoxiques du fluorure de benzalkonium. L'activité virucide est déterminée en fonction du temps de contact entre le fluorure de benzalkonium et la souche virale. Différents prélèvements sont effectués à intervalles de temps prédéterminés, puis soumis à une centrifugation pendant 8 minutes à 1 000 g, puis sont titrés sur microplaques. Une suspension virale témoin sans fluorure de benzalkonium est traitée suivant la même méthodologie. Les tableaux 2 à 5 fournissent les résultats obtenus en termes de cinétique d'inactivation des virus en fonction du temps de contact avec le fluorure de benzalkonium, et de sa concentration. L'activité virucide du fluorure de benzalkonium (FBK) est déterminée par la concentration minimale et le temps de contact minimal avec le virus qui provoquent une réduction du titre infectieux des virus d'un facteur logarithmique au moins égal à 4. Les essais ont été réalisés à 32° C.

**Tableau 2**

| ACTIVITE VIRUCIDE DU FBK SUR ENTEROVIRUS POLIO 1 Titre Viral (UFP/ml) | | | | |
|---|---|---|---|---|
| Concentration en FBK (%) (m/V) | Temps de contact virus/FBK (minutes) | | | |
| | 15 | 30 | 60 | 120 |
| 0,02 | 10^{3,28} | 10^{2,02} | 10^{1,40} | 0 |
| 0,01 | 10^{1,28} | 0 | 0 | 0 |
| 0,005 | 10^{4,38} | 10^{3,62} | 10^{3,02} | 10^{2,26} |

**Tableau 3**

| ACTIVITE VIRUCIDE DU FBK SUR ADENOVIRUS TYPE 5 Titre Viral (UFP/ml) | | | | |
|---|---|---|---|---|
| Concentration en FBK (%) (m/V) | Temps de contact virus/FBK (minutes) | | | |
| | 15 | 30 | 60 | 120 |
| 0,02 | 10^{5,88} | 10^{5,02} | 10^{4,16} | 10^{2,68} |
| 0,01 | 10^{5,04} | 10^{4,38} | 10^{3,86} | 10^{3,02} |
| 0,005 | 10^{6,00} | 10^{5,65} | 10^{5,10} | 10^{4,60} |

**Tableau 4**

| ACTIVITE VIRUCIDE DU FBK SUR HERPES SIMPLEX VIRUS TYPE 1 Titre Viral (UFP/ml) | | | | |
|---|---|---|---|---|
| Concentration en FBK (%) (m/V) | Temps de contact virus/FBK (minutes) | | | |
| | 15 | 30 | 60 | 120 |
| 0,02 | 0 | 0 | 0 | 0 |
| 0,01 | 10^{1,68} | 0 | 0 | 0 |
| 0,005 | 10^{4,26} | 10^{3,15} | 10^{2,27} | 10^{1,36} |

**Tableau 5**

| ACTIVITE VIRUCIDE DU FBK SUR HERPES SIMPLEX VIRUS TYPE 2 Titre Viral (UFP/ml) | | | | |
|---|---|---|---|---|
| Concentration en FBK (%) (m/V) | Temps de contact virus/FBK (minutes) | | | |
| | 15 | 30 | 60 | 120 |
| 0,02 | 0 | 0 | 0 | 0 |
| 0,01 | 10^{2,68} | 10^{1,41} | 0 | 0 |
| 0,005 | 10^{5,14} | 10^{4,65} | 10^{3,84} | 10^{2,26} |

Ces essais ont donc confirmé l'activité virucide du fluorure de benzalkonium aux concentrations (m/V) et temps de contact suivants : 0,01 % en 30 minutes sur entérovirus Polio 1 ; 0,02 % en 120 minutes sur adénovirus Type 5 ; 0,01 % en 5 minutes sur Herpès Simplex Virus Type 1 ; 0,01 % en 15 minutes sur Herpès Simplex Virus Type 2 à 32° C suivant la norme AFNOR T72-180.

### Exemple 2

L'activité virucide du fluorure de benzalkonium a été testée sur le virus VIH-1 (souche LAI) responsable du SIDA par l'Institut Pasteur, à l'aide du test d'infectivité sur culture primaire de lymphocytes humains activés.

L'étude a été réalisée en deux étapes : on a tout d'abord traité le virus avec une solution de fluorure de benzalkonium (FBK) à 20 µg/ml ; puis on a réalisé l'étude de l'infectivité résiduelle du virus traité sur culture primaire de lymphocytes humains activés.

Pour le traitement du virus, le temps de contact, à température ambiante, a été de 30 minutes et 60 minutes. 0,9 ml de virus additionné de 100 µl de FBK ont été laissés 30 minutes à température ambiante puis dilués dans 10 ml de tampon NTE. L'échantillon obtenu est référencé REV7. L'échantillon REV8 correspond à 0,9 ml de virus additionné de 100 µl de FBK laisses pendant 60 minutes à température ambiante puis dilués dans 10 ml de tampon NTE. Après dilution dans le tampon, les échantillons sont centrifugés 35 minutes à 40 000 tours/minutes afin d'éliminer le produit éventuellement toxique pour les cellules du test d'infectivité, et de concentrer le virus infectieux résiduel. Après centrifugation, le culot est resuspendu dans 1 ml de milieu de culture, aliquoté et conservé à -80° C.

Les échantillons REV1, REV2 et REV3.1 sont les témoins positifs de l'expérience. REV1 correspond à 0,9 ml de suspension virale congelée à -80° C. REV2 correspond à 0,9 ml de suspension virale diluée et centrifugée de la même façon que les échantillons REV7 et REV8, afin d'évaluer la perte d'infectivité pendant l'étape de centrifugation. REV3.1 correspond à 0,9 ml de suspension virale laissée à température ambiante pendant 60 minutes (durée maximale de l'étude) puis diluée en tampon NTE et centrifugée de la même façon que les échantillons traités, afin d'évaluer la perte d'infectivité due aux conditions expérimentales utilisées pour le FBK.

Un échantillon REV4 de 1 ml de FBK à 20 µg/ml dilué dans 10 ml de tampon NTE et centrifugé dans les conditions décrites pour le virus traité, est utilisé pour contrôler une éventuelle toxicité sur les cellules des tests d'infectivité qui serait due à des traces de produit résiduel non éliminé lors de la centrifugation. Cet échantillon est donc utilisé comme témoin négatif avec les cellules du test d'infectivité non infectées.

Les essais proprement dits ont constitué à rechercher le pouvoir infectieux résiduel des échantillons de virus traité sur les lymphocytes PBMC humains activés. Pour ce faire, les lymphocytes préalablement stimulés sont incubés avec 1 ml de chaque échantillon de virus traité ou non, dilué de 10 en 10 (de 10⁻⁴ à 10⁻⁷ pour les témoins positifs, et de 10⁻¹ à 10⁻⁵ pour les échantillons de virus traité). Les cellules sont centrifugées et resuspendues à 10⁶ cellules par ml dans du milieu complet. Tous les trois ou quatre jours, les cellules sont comptées, ré-ensemencées et les surnageants conservés à -80° C. Après analyse des résultats, une deuxième expérience a été réalisée de façon identique en inoculant les cellules avec des dilutions moitié des échantillons de virus traité par le FBK.

Ensuite, on a mesuré l'activité transcriptase inverse (RT) dans les surnageants de culture cellulaire. Les surnageants conservés à chaque passage cellulaire (jours n° 3, 7, 10, 14, 17, 21, 24 pour la première expérience et 3, 6, 9, 13, 16 et 20 pour la deuxième expérience), ont été testés, et leur activité transcriptase inverse a été dosée après concentration par ultracentrifugation.

On a aussi réalisé des dosages de l'antigène P25 du VIH-1 à l'aide de la technique Elisa "Sandwich" sur les mêmes surnageants de culture aux jours n° 3, 10, 17 et 24 pour la première expérience et 3, 9, 16 et 20 pour la deuxième expérience après inoculation des cellules. Le kit ELAVIA Ag1 commercialisé par la Société Diagnostics Pasteur a été utilisé. On a constaté que les échantillons de contrôle REV4 ne sont pas toxiques pour les cellules du test d'infectivité.

Le tableau 6 fournit les résultats obtenus de l'activité reverse transcriptase pour les échantillons témoins.

**Tableau 6**

| TEST DE L'ACTIVITE VIRUCIDE DU FBK SUR CULTURE PRIMAIRE DE LYMPHOCYTES HUMAINS ACTIVES : TITRAGE EN DILUTIONS DES ECHANTILLONS DE VIRUS TEMOINS | | | | | | | | |
|---|---|---|---|---|---|---|---|---|
| Echantillon | Dilution | Activité RT aux jours (cpm/ml*) | | | | | | |
| | | J3 | J7 | J10 | J14 | J17 | J21 | J24 |
| REV1 témoin positif | 10⁻⁴ | 391 | 4 304 | 26 311 | 47 192 | 5 529 | 888 | 712 |
| | 10⁻⁵ | 1 354 | 1 786 | 5 417 | 36 738 | 8 450 | 7 157 | 3 854 |
| | 10⁻⁶ | 335 | 652 | 2 459 | 38 850 | 23 062 | 5 611 | 3 368 |
| | 10⁻⁷ | 490 | 1 121 | 6 521 | 5 940 | 2 313 | 1 014 | 1 370 |
| REV2 témoin centrifugation | 10⁻⁴ | 285 | 590 | 1 345 | 9 772 | 4 075 | 1 888 | 2 083 |
| | 10⁻⁵ | 713 | 294 | 296 | 555 | 9 348 | 12 302 | 5 043 |
| | 10⁻⁶ | 308 | 351 | 414 | 326 | 3 509 | 1 568 | 653 |
| REV3.1 témoin expérimental 60 min | 10⁻⁴ | 203 | 582 | 569 | 8 425 | 3 703 | 3 778 | 4 559 |
| | 10⁻⁵ | 572 | 505 | 577 | 1 395 | 3 155 | 12 243 | 4 607 |
| | 10⁻⁶ | 440 | 339 | 1 165 | 1 250 | 714 | 653 | 994 |
| cellules non infectées | | 247 | 258 | 363 | 291 | 630 | 568 | 366 |

| | | | | | | | | |
|---|---|---|---|---|---|---|---|---|
| *cpm/ml : activité reverse transcriptase (RT) dans 1 ml de surnageant de culture. La production virale est considérée positive pour une activité RT supérieure à 5 000 cpm/ml (chiffres en gras) | | | | | | | | |

On constate sur le tableau 6 que 14 jours après l'infection, les cellules inoculées avec des dilutions inférieures ou égales à 10⁻⁷ de l'échantillon REV1 ou avec la dilution 10⁻⁴ pour les échantillons REV2 et REV3.1 expriment des particules virales dans les surnageants de culture. Dans les conditions expérimentales, le titre du virus était défini comme l'inverse de la dernière dilution infectieuse à 100 %. Les titres des échantillons témoins sont respectivement de 10⁻⁷ UI/ml pour REV1 et 10⁴ UI/ml pour REV2 et REV3.1. Une diminution du titre infectieux est donc constatée pour les témoins expérimentaux. Elle est d'un facteur logarithmique de l'ordre de 3 pour le témoin de centrifugation, et pour le virus témoin laissé 1 heure à température ambiante et centrifugé. Sur la figure 1, on constate qu'aucune activité reverse transcriptase n'est décelée dans les surnageants des cultures inoculées avec une dilution 1/10 des échantillons de virus traités par le FBK pendant 30 ou 60 minutes. Ces résultats sont à comparer à ceux obtenus avec le témoin REV3.1 qui présente un titre infectieux de 10⁴ UI/ml.

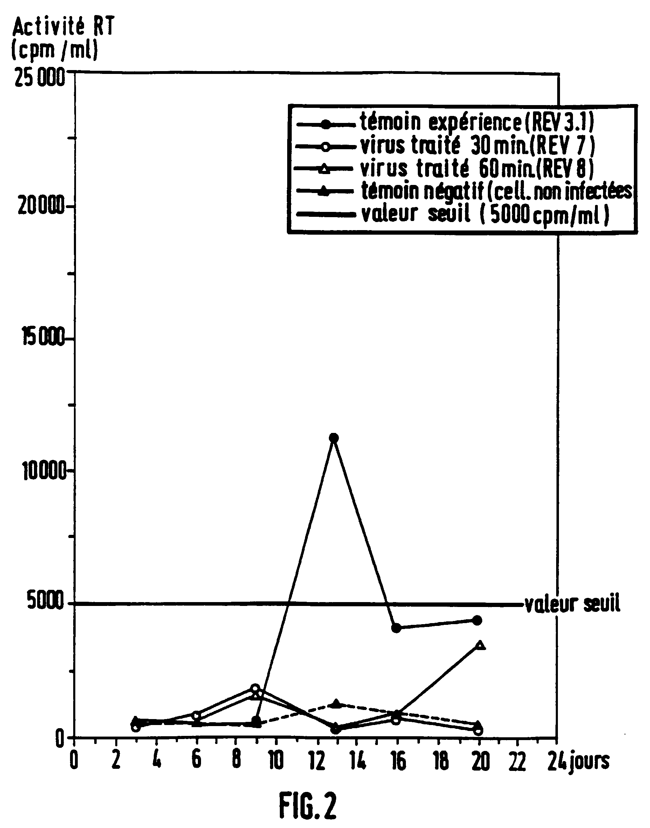

Compte tenu de ces résultats, une deuxième expérience a été effectuée en inoculant des lymphocytes PBMC avec des dilutions 1/2 des échantillons de virus traités par FBK afin de confirmer l'absence de virus infectieux dans les échantillons. Les résultats des dosages de l'activité RT dans les surnageants de culture sont représentés sur la figure 2. Aucune activité RT n'est détectée alors que l'échantillon de virus témoin REV3.1 s'est avéré positif à la dilution 10⁻⁵.

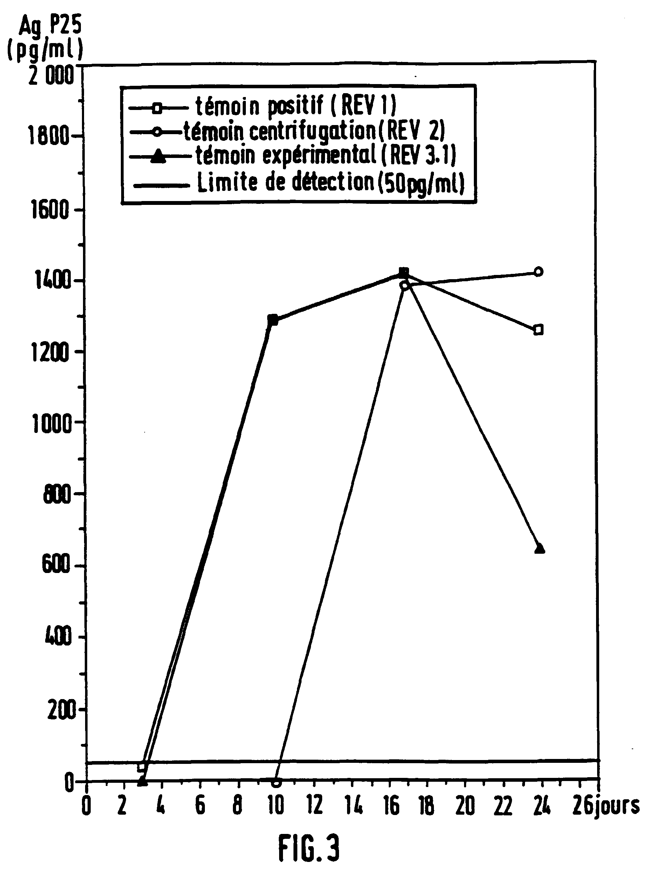

La figure 3 représente les résultats des dosages de l'antigène P25 dans les échantillons de virus témoins. La quantité d'antigène P25 est exprimée en pg/ml et est évaluée à l'aide d'une courbe étalon établie à partir d'une solution d'antigènes de référence fournie par le fabricant. Les titres évalués 16 jours après l'infection sont de 10⁷ UI/ml pour REV1, 10⁵ UI/ml pour REV2 et 10⁴ UI/ml pour REV3.1.

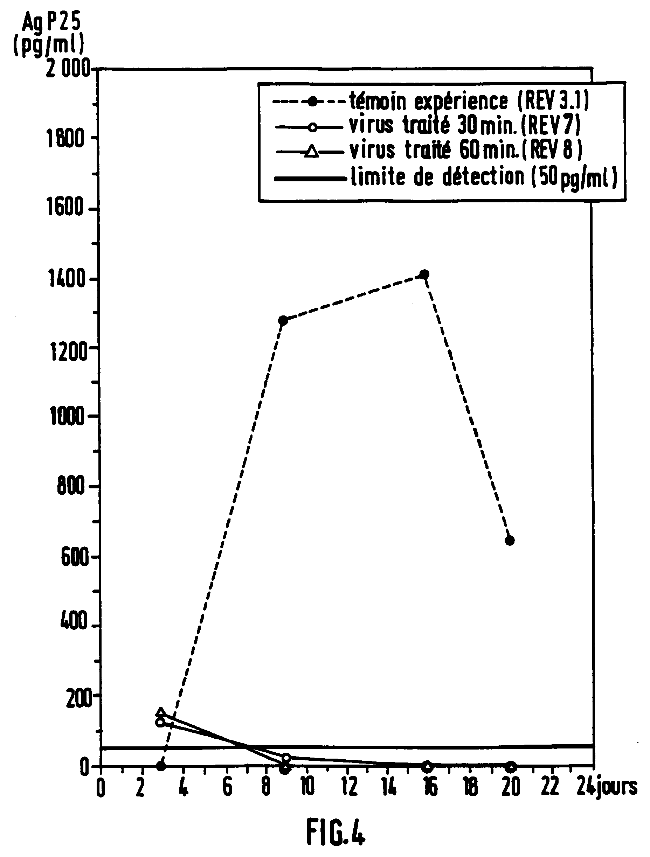

La figure 4 donne le dosage en antigènes P25 sur les surnageants de lymphocytes inoculés avec des dilutions 1/2 des échantillons de virus traités par FBK. On constate que les échantillons REV7 et REV8 sont négatifs en antigène P25. Ces résultats confirment donc et précisent ceux obtenus en suivant l'expression virale par dosage de l'activité reverse transcriptase dans les surnageants des cultures exposées à du virus traité ou non.

Ainsi, ces essais démontrent que le fluorure de benzalkonium à 20 µg/ml induit une perte du pouvoir infectieux du virus VIH-1 in vitro d'un facteur logarithmique de 5 pour des lymphocytes humains activés. Ainsi, le fluorure de benzalkonium à 20 µg/ml s'est avéré virucide sur le virus du SIDA in vitro.

### Exemple 3

La tolérance locale et générale de l'administration du fluorure de benzalkonium par voie intraveineuse a été testée chez le chien.

Deux chiens beagle, un mâle et une femelle, d'un poids initial voisin de 10 kg, âgés de 6 mois, en bonne santé, déparasités et vaccinés, ont été placés en animalerie. Ils ont subi un traitement par voie intraveineuse de 2 mg par kg et par jour de fluorure de benzalkonium dilué à 2,10 g/l dans du soluté isotonique de chlorure de sodium apyrogène stérile. La solution est conditionnée en ampoules de 10 ml et on a injecté 0,95 ml par kg et par jour en effectuant une rotation des injections sur la veine de chaque membre. L'injection est réalisée à l'aide d un micro-perfuseur épicrânien monté sur une seringue plastique, et l'administration est réalisée en intraveineuse lente d'une minute trente secondes environ. Le traitement a été réalisé pendant 28 jours. Des examens urinaires ont été pratiqués avant le début du traitement, au milieu (après 14 jours) et à la fin du traitement.

Egalement, un prélèvement de sang est effectué à la jugulure avant le début du traitement, après 14 jours, et après les 28 jours de traitement. On a réalisé sur ce sang les examens hématologiques et des dosages biochimiques complets.

A la fin du traitement, les animaux ont été sacrifiés, et on a procédé à des examens anatomopathologiques et à une pesée des organes suivants : foie, reins, rate, surrénales, testicules, ovaires, thymus, thyroïdes. On a procédé également à des examens histologiques sur la plupart des organes vitaux.

Aucune modification du comportement symptomatologique n'a été observée à la suite du traitement. Pendant les deux premières semaines, les injections ont pu être réalisées sans difficultés. Par la suite, un oedème est apparu.

Le poids corporel des animaux est resté stable pendant toute la durée de l'expérimentation. La température rectale du chien mâle est restée stable et celle du chien femelle a augmenté de 1° C au cours de l'expérimentation. Aucune variation notable de pression sanguine et de fréquence cardiaque n'a pu être observée. Egalement, les différents paramètres électrocardiographiques sont restés stables pendant toute la durée de l'expérimentation.

Pour le chien femelle, les examens urinaires n'ont décelé aucune trace d'hémolyse, au début, au milieu et à la fin du traitement. Pour le chien mâle, on a constaté une forte présence de sang dans l'urine au début du traitement. Le chien a été laissé dans une cage à diurèse pendant 48 heures. A la fin des 48 heures, l'analyse urinaire a démontré l'absence de sang, des traces de corps cétonique, l'absence de glucose et des traces de protéines. Les dosages du sodium et du potassium ont subi des variations pour le chien mâle mais sont restées quasiment stables pour le chien femelle. A la fin du traitement, aucune trace de sang dans l'urine prélevée dans la vessie du chien mâle au moment de l'autopsie, n'a été constatée.

Parmi les paramètres hématologiques, seul le nombre de leucocytes a augmenté après deux semaines pour le chien femelle et après quatre semaines pour le chien mâle. Cette augmentation correspond à l'apparition d'oedèmes à une patte.

Dans les paramètres biochimiques, une augmentation des phosphatases alcalines et des LDH a pu être constatée. Aucune autre variation significative n'a été constatée.

L'examen histologique des différents organes n'a pas pu montrer d'altération due au traitement, si ce n'est les réactions locales sur les sites d'injection. Ainsi, malgré la sévérité du traitement, aucun effet toxique remarquable du fluorure de benzalkonium injecté par voie intraveineuse n'a pu être mis en évidence.

### Exemple 4

La toxicité aiguë du fluorure de benzalkonium en administration unique chez la souris et le cobaye a été déterminée par la méthode de Behrens et Karber, par voie intracardiaque, intraveineuse, et intrapéritonéale. Les animaux utilisés étaient des souris albinos et des cobayes albinos adultes jeunes. Dix animaux sont utilisés par dose administrée, cinq femelles et cinq mâles. Le fluorure de benzalkonium a été utilisé en solution à 3 g/l dans du soluté isotonique de chlorure de sodium apyrogène stérile. On a observé les animaux durant 7 jours après l'administration unique réalisée à l'aide d'une aiguille stérile adaptée à une seringue graduée en dixièmes de millitres pour permettre de traiter chaque animal avec une dose exactement connue. Les animaux sont pesés tous les jours pendant la durée de l'observation.

La DL 50 (dose létale 50) a été déterminée. Les résultats sont donnés dans le tableau 7.

**Tableau 7**

| DL 50 (mg/kg) | voie intraveineuse | voie intrapéritonéale | voie intracardiaque |
|---|---|---|---|
| Souris mâle | 33,24 | 33,1 | - |
| Souris femelle | 36,6 | 34,6 | - |
| Cobaye mâle | - | 28,5 | 15,5 |
| Cobaye femelle | - | 20,5 | 16,5 |

Ainsi, on constate que le fluorure de benzalkonium par voie intraveineuse, intrapéritonéale ou intracardiaque est suffisamment peu toxique pour que l'on puisse envisager son administration par voie intravasculaire à des doses où il est néanmoins actif, notamment de l'ordre de 20 à 23 mg par litre de sang du patient à traiter.

### Exemple 5

Des essais in vivo ont été réalisés sur des chiens atteints de parvovirose. Ces chiens ont été traités en perfusion lente par du fluorure de benzalkonium C14 dissous à 2,1 g/l dans du soluté isotonique de chlorure de sodium apyrogène stérile.

Un Berger Allemand de 20 kg mâle âgé de huit mois atteint de parvovirose (gastro-entérite avec déshydratation, entérite séro-hémorragique avec fort taux d'urée) a été traité par deux injections à 12 heures d'intervalle de deux ampoules de 10 ml. On a constaté 6 jours après le début du traitement, à la fois une excellente tolérance et un rétablissement du chien.

Un Berger Allemand femelle âgé de 7 mois et d'un poids de l'ordre de 10 kg, malade de parvovirose depuis 4 jours environ (gastro-entérite avec déshydratation, état sub-comateux, hémoconcentration, fort taux d'urée, sel hémorragique) a été traité avec deux injections séparées de 12 heures de solution selon l'invention. La première injection a été de 10 ml, et la deuxième a été de 20 ml. Un traitement général associé a été effectué pendant 8 jours. La tolérance du traitement s'est avérée excellente. Malgré l'état très mauvais du chien au début du traitement, on a constaté un rétablissement total au bout de 20 jours.

Un chien mâle Terre-Neuve de 6 mois et d'un poids d'environ 28 kg atteint de parvovirose a également été traité avec deux injections séparées de 12 heures de 10 ml par voie intraveineuse lente. Au bout du deuxième jour, le sang a disparu des urines et le chien ne vomissait plus. Au bout du quatrième jour, l'animal a repris son alimentation. Au 18ème jour, un rétablissement complet a été constaté.

Un chien femelle Rotweiler âgé de 4 mois et d'un poids de 15 kg atteint de parvovirose a également été traité de la même façon. Un rétablissement complet a été constaté au bout de 18 jours.

Le même traitement a été appliqué à un chien femelle Braque Allemand âgé de 2 mois, d'un poids d'environ 12 kg. Le même rétablissement a été constaté (reprise alimentaire au 4ème jour et rétablissement total confirmé au 18ème jour).

Trois chiots (mâle de 4 mois et de 12 kg ; mâle de 4 mois et de 7 kg ; femelle de 2 mois et de 6 kg) atteints de gastro-entérite violente évolutive ont également été traités par intraveineuse lente avec deux injections à 12 heures d'intervalle. Les chiots étaient atteints de gastro-entérite séro-hémorragique. La mort a été constatée dans les deux jours.

Ainsi, à l'exception des trois échecs obtenus sur des animaux très jeunes et atteints de symptômes sévères, l'invention a permis de traiter des parvoviroses du chien.

### Exemple 6

L'invention a été utilisée pour traiter des chiens atteints de la maladie de Carré. Cette maladie, considérée comme mortelle et sans traitement à ce jour, est due à un ultravirus.

Douze chiens ont été traités à raison d'une ampoule de 10 ml de solution de fluorure de benzalkonium à 2,1 g/l pour 20 kg d'animal. L'injection est réalisée une fois par jour pendant trois jours. Les chiens ont subi des traitements associés (vitamines, antibiotiques, vaccins...).

Le traitement s'est avéré efficace sur sept chiens qui ont pu être sauvés et pour lesquels on a constaté un rétablissement total au bout de 18 jours.

Parmi les sept chiens guéris, cinq d'entre eux provenaient d'une meute dans laquelle deux décès avaient déjà été constatés, et étaient très atteints par la maladie. Ils avaient déjà subi un premier traitement inefficace. Les symptômes étaient notamment des écoulements purulents aux narines, toux, diarrhées, conjonctivite suppurée, fièvre comprise entre 40° C et 40,5° C en moyenne. L'efficacité du traitement a donc été particulièrement surprenante.

Les cinq autres chiens traités présentaient tous des symptômes nerveux (tremblement généralisé) et n'ont pas pu être soignés. En conséquence, l'invention permet le traitement des cas de maladie de Carré non accompagnés de manifestations nerveuses.

### Exemple 7

On a déterminé in vitro la concentration hémolytique du fluorure de benzalkonium sur du sang total humain, de cobaye et de lapin.

Cette expérimentation a été effectuée avec un mode opératoire hospitalier de recherche d'une anémie périphérique. On a utilisé des dilutions d'une solution de fluorure de benzalkonium à 3 pour mille : 300 mg/l ; 150 mg/l et 75 mg/l dans du soluté isotonique de chlorure de sodium apyrogène stérile. Chacune des trois dilutions est mélangée à volume égal avec du sang des trois espèces (homme, cobaye et lapin). Le mélange est agité lentement pendant 5 minutes trente puis centrifugé trois minutes à 3 500 tours/minute. L'hémolyse se traduit par une coloration rouge plus ou moins foncée du surnageant plasmatique. Les concentrations réelles testées ont donc été de 150 ppm, 75 ppm et 35,5 ppm de fluorure de benzalkonium. Chaque essai a été doublé.

N'ayant pas constaté d'hémolyse à 37,5 mg/l, de nouveaux essais ont été réalisés sur six échantillons de sang humain, dans les mêmes conditions, à 20 mg/l et 35 mg/l de fluorure de benzalkonium.

Le tableau 8 ci-après donne les résultats obtenus.

**Tableau 8**

| | Concentrations on fluorure de benzalkonium (en ppm ou mg/l) | | | | |
|---|---|---|---|---|---|
| | | | | 6 échantillons | |
| | 150 | 75 | 37,5 | 35 | 20 |
| COBAYE | ++++ | + | 0 | - | - |
| LAPIN | +++ | + | 0 | - | - |
| HOMME | ++ | + | 0 | 0 | 0 |
| + à ++++ : Degré de coloration rouge due à l'hémolyse | | | | | |

Ainsi, le fluorure de benzalkonium à une concentration égale ou inférieure à 20 mg/l ne provoque pas d'hémolyse du sang humain, et ce avec une bonne marge de sécurité.

Selon l'invention, on peut néanmoins vérifier aisément avant l'injection la concentration hémolytique du fluorure de benzalkonium sur le sang du patient à traiter.

### Exemple 8

On a traité des malades atteints de SIDA en phase évolutive ou terminale par une solution comprenant 2,1 g/l de fluorure de benzalkonium. Avant l'injection, la concentration hémolytique du fluorure de benzalkonium a été déterminée, et on a vérifié qu'elle était largement supérieure à celle de la solution utilisée.

Un homme âgé de 37 ans, pesant 70 kg, présentait les symptômes suivants : asthénie, amaigrissement, candidose buccale, fièvre en plateau à 39,7° C, tests ELISA ("enzyme linked immunosorbent assay") et WESTERN BLOT (test de confirmation) positifs, nombre de CD₄ (récepteurs à la surface des lymphocytes T responsables de la régulation de la réponse immunitaire) inférieur à 200 (la valeur normale étant de l'ordre de 1 000). Le patient a été traité par des injections intraveineuses lentes de 50 ml de fluorure de benzalkonium C₁₄ à 2,1 g/l répétées toutes les douze heures, en deux cures de trois jours espacées de cinq jours d'observation. Malgré une douleur au point d'injection apparue à la fin de la deuxième cure, les observations à un mois et à sept mois après le traitement démontrent un bon état général du patient qui a pu reprendre son activité. Le nombre des CD₄ est remonté à 600.

Un homme âgé de 28 ans, pesant 57 kg, présentait les symptômes suivants : asthénie, amaigrissement, vomissements, diarrhées, candidose buccale, fièvre en plateau à 39,5° C, malade totalement grabataire, tests ELISA et WESTERN BLOT positifs, nombre de CD₄ de 20. Ce patient a subi deux cures de trois jours comme mentionné précédemment. Au dixième jour après le début du traitement, on note une amélioration nette de l'état général, disparition de la fièvre, des vomissements et des diarrhées et une reprise de poids (59,6 kg). Le malade peut se lever et marcher.

Un homme âgé de 44 ans, pesant 78 kg, présentait les symptômes suivants : asthénie, anorexie, insomnies, fièvre en plateau à 39,7° C, tests ELISA et WESTERN BLOT positifs, nombre de CD₄ de 650. Le patient a été traité par des injections intraveineuses lentes de 50 ml de fluorure de benzalkonium C₁₄ à 2,1 g/l répétées toutes les huit heures pendant trois jours avec un traitement complémentaire antibiotique (à base de Doxycycline) de deux gélules chaque soir.

Au sixième jour après le début du traitement, la fièvre a disparu. Deux mois après le début du traitement, on note une disparition progressive de l'asthénie. Au quatrième mois, le patient revenu à un état général normal a repris son activité militaire.

Un homme agé de 42 ans, pesant 52 kg, présentait les symptômes suivants : asthénie, anorexie, sueurs, adénopathie inguinale, diarrhées, céphalées, candidose buccale, fièvre en plateau de 39° C, tests ELISA et WESTERN BLOT positifs, nombre de CD₄ de 200. Le patient a été traité par une cure de trois jours de fluorure de benzalkonium C₁₄ à 2,1 g/l en injections intraveineuses lentes toutes les douze heures. Dès le troisième jour, une nette chute de la fièvre est constatée. Trois mois et demi après le début du traitement, le patient présente un bon état général, un poids de 56 kg, un nombre de CD₄ remonté à 700 et aucun symptôme clinique.

### Exemple 9

On a déterminé l'activité in vitro du fluorure de benzalkonium sur le mycobacterium tuberculosis (souche sauvage).

Cette expérimentation a été effectuée avec le mode opératoire d'imprégnation de la surface du milieu BOWEINSTEIN JENSEN. La quantité de substance active absorbée par le mileu est exprimée en cm².

Le volume utilisé de la dilution appropriée du fluorure de benzalkonium était de 0,25 ml par tube de milieu afin d'arriver à des concentrations de fluorure de benzalkonium de 0,1, 0,2 et 0,3 % par surface en cm² de BOWEINSTEIN JENSEN.

Un tube témoin, sans fluorure de benzalkonium, a été utilisé pour chacuns des séries.

Trois séries de tests ont été réalisées avec des dilutions de 0,1, 0,01, et 0,001 de solution mère de la souche Bacterium Tuberculosis préparée à 1 mg/ml.

Tous les tests ont été effectués en double.

Après quatre semaines d'incubation à 37°C, les résultats montrent une inhibition de culture avec les concentrations en fluorure de benzalkonium de 0,1, 0,2 et 0,3 % par surface en cm² de milieu BOWEINSTEIN JENSEN et ceci a été observé pour les trois dilutions à 0,1, 0,01 et 0,001 de la culture mère de Bacterium Tuberculosis.

Le tableau 9 ci-après donne les résultats obtenus.

**Tableau 9**

| | BK 0,1 | BK 0,1 | BK 0,01 | BK 0,01 | BK 0,001 | BK 0,001 |
|---|---|---|---|---|---|---|
| TEMOIN | (+++) | (+++) | (+++) | (+++) | (+++) | (+++) |
| FBK 0,1%/cm² | (-) | (-) | (-) | (-) | (-) | (-) |
| FBK 0,2%/cm² | (-) | (-) | (-) | (-) | (-) | (-) |
| FBK 0,3%/cm² | (-) | (-) | (-) | (-) | (-) | (-) |

| | | | | | | |
|---|---|---|---|---|---|---|
| (+++) = culture positive | | | | | | |
| (-) = culture négative | | | | | | |

## Revendications

1. Solution administrable par voie intravasculaire caractérisée en ce qu'elle comprend de l'ordre de ou moins de 0,23 % en poids d'au moins un fluorure de benzalkonium de formule : 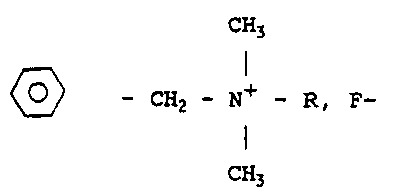 ou R est un radical alcoyle pouvant varier entre C₈H₁₇ et C₁₈H₃₇ , dissous dans un excipient injectable par voie intravasculaire.

2. Solution selon la revendication 1 caractérisée en ce qu'elle comprend de l'ordre de 0,2 % en poids de fluorure de benzalkonium.

3. Solution selon l'une des revendications 1 et 2, caractérisé en ce qu'elle comprend plus de 0,05 % en poids de fluorure de benzalkonium.

4. Solution selon l'une des revendications 1 à 3, caractérisée en ce qu'elle comprend une concentration de fluorure de benzalkonium déterminée pour pouvoir administrer en injection lente des doses de l'ordre de 0,5 à 23 mg - notamment de l'ordre de 21 mg - de fluorure de benzalkonium par litre de sang du patient à traiter.

5. Solution selon l'une des revendications 1 à 4, caractérisée en ce que R est compris entre C₁₂H₂₅ et C₁₄H₂₉.

6. Solution selon l'une des revendications 1 à 5, caractérisée en ce qu'elle comprend de 0,05 à 2,3 g/l - notamment de l'orde de 2,1 g/l- de fluorure de benzalkonium où R est C₁₄H₂₉

7. Solution selon l'une des revendications 1 à 6, caractérisée en ce qu'elle est conditionnée en ampoules de 5 à 15 ml -notamment de 10ml- comprenant entre 0,5 et 23 mg -notamment de l'ordre de 21 mg- de fluorure de benzalkonium.

8. Solution selon l'une des revendications 1 à 7, caractérisée en ce qu'elle est constituée de fluorure de benkalkonium dissous dans un soluté physiologique.

9. Solution selon l'une des revendications 1 à 8, caractérisée en ce qu'elle comporte en outre au moins un dérivé métallique du fluor.

10. Solution selon l'une des revendications 1 à 9, caractérisée en ce qu'elle comporte en outre du fluorure de lithium.

11. Application d'au moins un fluorure de benzalkonium de formule 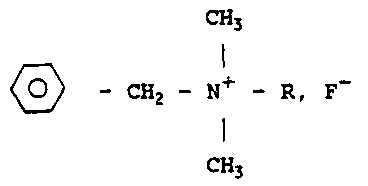 où R est un radical alcoyle pouvant varier entre C₈H₁₇ et C₁₈H₃₇ dissous à une concentration de l'ordre de ou intérieure à 0,23 % en poids dans un excipient injectable par voie intravasculaire pour obtenir une solution administrable par voie intravasculaire destinée à lutter contre les maladies virales ou infectieuses, notamment d'origine immunodéficitaire.

12. Application d'au moins un fluoruure de benzalkonium de formule 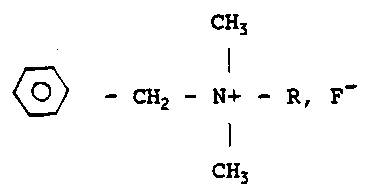 où R est un radical alcoyle pouvant varier entre C₈H₁₇ et C₁₈H₃₇ dissous à une concentration de l'ordre de ou inférieure à 0,23 % en poids dans un excipient injectable par voie intravasculaire pour obtenir une solution administrable par voie intravasculaire destinée à lutter contre le SIDA en phase déclarée ou évolutive.

13. Application d'au moins un fluoruure de benzalkonium de formule 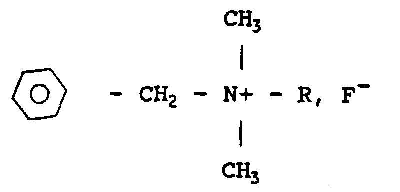 où R est un radical alcoyle pouvant varier entre C₈H₁₇ et C₁₈H₃₇ dissous à une concentration de l'ordre de ou inférieure à 0,23 % en poids dans un excipient injectable par voie intravasculaire pour obtenir une solution administrable par voie intravasculaire destinée à lutter contre la tuberculose.

14. Application selon l'une des revendications 11 à 13 caractérisée en ce qu'on utilise de l'ordre de 0,2 % en poids de fluorure de benzalkonium.

15. Application selon l'une des revendications 11 à 13 caractérisée en ce qu'on utilise plus de 0,05 % en poids de fluorure de benzalkonium.

16. Application selon l'une des revendications 11 à 15 caractérisée en ce que la concentration de fluorure de benzalkonium est déterminée pour pouvoir administrer en injection lente des doses de l'ordre de 0,5 à 23 mg - notammant de l'ordre de 21 mg- de fluorure de benzalkonium par litre de sang du patient à traiter.

17. Application selon l'une des revendications 11 à 14, caractérisée en ce que R est compris entre C₁₂H₂₅ et C₁₄H₂₉.

18. Application selon l'une des revendications 12 à 17, caractérisée en ce qu'on utilise entre 0,5 et 23 mg - notamment de l'ordre de 21 mg- de fluorure de benzalkonium dissous dans 10 ml de soluté.

19. Application selon l'une des revendications 11 à 18, caractérisée en ce qu'au moins un fluorure de benzalkonium est dissous dans un soluté physiologique.

20. Application selon l'une des revendications 11 à 19, caractérisée en ce qu'on utilise également au moins un dérivé métallique du fluor.

21. Application selon la revendication 20, caractérisée en ce qu'on utilise également du fluorure de lithium.

## Claims

1. A solution for intravascular administration characterised in that it comprises about 0.23% or less by weight of at least one benzalkonium fluoride having the formula: 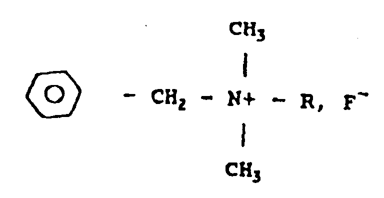 where R is an alkyl radical which can vary between C₈H₁₇ and C₁₈H₃₇, dissolved in an excipient injectable intravascularly.

2. A solution according to claim 1, characterised in that it contains about 0.2% by weight of benzalkonium fluoride.

3. A solution according to claim 1 or 2, characterised in that it contains more than 0.05% by weight of benzalkonium fluoride.

4. A solution according to any of claims 1 to 3, characterised in that it contains a concentration of benzalkonium fluoride suitable for administration by slow injection of doses of about 0.5 to 23 mg, particularly about 21 mg, of benzalkonium fluoride per litre of the patient's blood.

5. A solution according to any of claims 1 to 4, characterised in that R is between C₁₂H₂₅ and C₁₄H₂₉.

6. A solution according to any of claims 1 to 5, characterised in that it contains 0.05 to 2.3 g/l, particularly about 2.1 g/l, of benzalkonium fluoride where R is C₁₄H₂₉.

7. A solution according to any of claims 1 to 6, characterised in that it is packed in 5 to 15 ml, particularly 10 ml, ampoules containing between 0.5 and 23 mg, particularly about 21 mg, of benzalkonium fluoride.

8. A solution according to any of claims 1 to 7, characterised in that it comprises benzalkonium fluoride dissolved in a physiological solute.

9. A solution according to any of claims 1 to 8, characterised in that it also contains at least one metal fluorine derivative.

10. A solution according to any of claims 1 to 9, characterised in that it also contains lithium fluoride.

11. Use of at least one benzalkonium fluoride having the formula 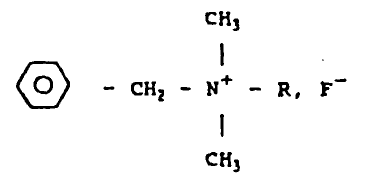 where R is an alkyl radical which can vary between C₈H₁₇ and C₁₈H₃₇ dissolved at a concentration of about 0.23% or less by weight in an excipient injectable intravascularly in order to obtain a solution for intravascular administration and intended for use against viral or infectious diseases, particularly of immunodeficit origin.

12. Use of at least one benzalkonium fluoride having the formula 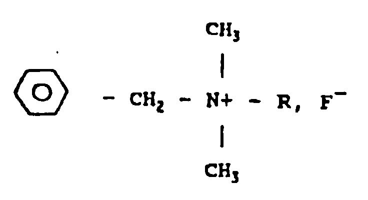 where R is an alkyl radical which can vary between C₈H₁₇ and C₁₈H₃₇ dissolved at a concentration of about 0.23% or less by weight in an excipient injectable intravascularly in order to obtain a solution for intravascular administration and intended for use against AIDS in the declared or evolving phase.

13. Use of at least one benzalkonium fluoride having the formula 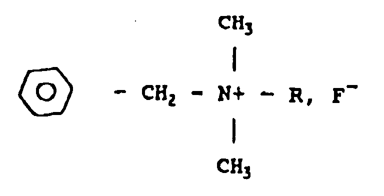 where R is an alkyl radical which can vary between C₈H₁₇ and C₁₈H₃₇ dissolved at a concentration of about 0.23% or less by weight in an excipient injectable intravascularly in order to obtain a solution for intravascular administration and intended for use against tuberculosis.

14. Use according to any of claims 11 to 13, characterised in that about 0.2% by weight of benzalkonium fluoride is used.

15. Use according to any of claims 11 to 13, characterised in that more than 0.05% by weight of benzalkonium fluoride is used.

16. Use according to any of claims 11 to 15, characterised in that the concentration of benzalkonium fluoride is made suitable for administration by slow injection of doses of the order of 0.5 to 23 mg, particularly about 21 mg, of benzalkonium fluoride per litre of the patient's blood.

17. Use according to any of claims 11 to 14, characterised in that R is between C₁₂H₂₅ and C₁₄H₂₉.

18. Use according to any of claims 12 to 17, characterised in that between 0.5 and 23 mg, particularly about 21 mg, of benzalkonium fluoride dissolved in 10 ml of solute is used.

19. Use according to any of claims 11 to 18, characterised in that at least one benzalkonium fluoride is dissolved in a physiological solute.

20. Use according to any of claims 11 to 19, characterised in that at least one metal fluorine derivative is also used.

21. Use according to claim 20, characterised in that lithium fluoride is also used.

## Patentansprüche

1. Intravaskulär zu verabreichende Lösung, dadurch gekennzeichnet, daß sie wenigstens ein Benzalkoniumfluorid in der Größenordnung von oder weniger als 0,23 Gew.% der Formel umfaßt: 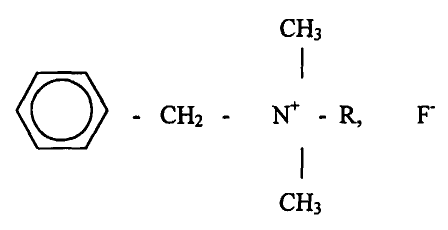 wobei R ein Alkylrest ist, der zwischen C₈H₁₇und C₁₈H₃₇ variieren kann, in einem intravaskulär injzierbaren Arzneimittelträger gelöst.

2. Lösung nach Anspruch 1, dadurch gekennzeichnet, daß sie in der Größenordnung von 0,2 Gew.% Fluorbenzalkonium umfaßt.

3. Lösung nach einem der Ansprüche 1 und 2, dadurch gekennzeichnet, daß sie mehr als 0,05 Gew.% Benzalkoniumfluorid umfaßt.

4. Lösung nach einem der Ansprüche 1 bis 3, dadurch gekennzeichnet, daß sie eine Konzentration Benzalkoniumfluorid umfaßt, die bestimmt ist, mit langsamer Injektion Dosen der Größenordnung von 0,5 bis 23 mg - insbesondere der Größenordnung von 21 mg - Benzalkoniumfluorid pro Liter Blut des zu behandelnden Patienten verabreichen zu können.

5. Lösung nach einem der Ansprüche 1 bis 4, dadurch gekennzeichnet, daß R von C₁₂H₂₅ und C₁₄H₂₉ umfaßt ist.

6. Lösung nach einem der Ansprüche 1 bis 5, dadurch gekennzeichnet, daß sie von 0,05 bis 2,3 g/L - insbesondere der Größenordnung von 2,1 g/L - Benzalkoniumfluorid umfaßt, wobei R C₁₄H₂₉ ist.

7. Lösung nach einem der Ansprüche 1 bis 6, dadurch gekennzeichnet, daß sie in Ampullen zu 5 bis 15 mL - insbesondere von 10 mL - konfektioniert ist, die zwischen 0,5 und 23 mg - insbesondere in der Größenordnung von 21 mg - Benzalkoniumfluorid umfassen.

8. Lösung nach einem der Ansprüche 1 bis 7, dadurch gekennzeichnet, daß sie aus Benzalkoniumfluorid besteht, das in einer physiologischen Lösung gelöst ist.

9. Lösung nach einem der Ansprüche 1 bis 8, dadurch gekennzeichnet, daß sie überdies wenigstens ein Metallderivat von Fluor umfaßt.

10. Lösung nach einem der Ansprüche 1 bis 9, dadurch gekennzeichnet, daß sie überdies Lithiumfluorid umfaßt.

11. Verwendung wenigstens eines Benzalkoniumfluorids der Formel 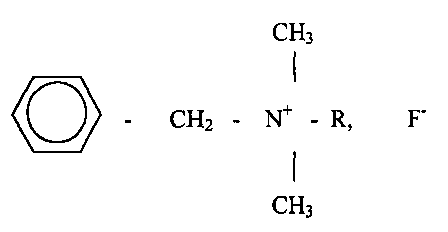 wobei R ein Alkylrest ist, der zwischen C₈H₁₇ und C₁₈H₃₇ variieren kann, zu einer Konzentration der Größenordnung von oder unterhalb von 0,23 Gew.% in einem intravaskulär injizierbaren Arzneimittelträger gelöst, um eine intravaskulär zu verabreichende Lösung zu erhalten, die bestimmt ist, virale oder infektiöse Krankheiten zu bekämpfen, insbesondere solche mit einem Ursprung in der Immunschwäche.

12. Verwendung wenigstens eines Benzalkoniumfluorids der Formel 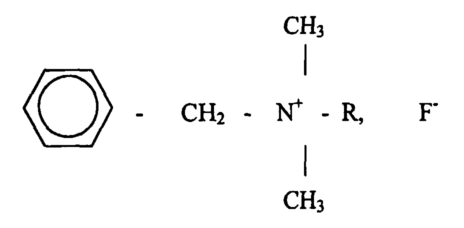 wobei R ein Alkylrest ist, der zwischen C₈H₁₇ und C₁₈H₃₇ variieren kann, zu einer Konzentration der Größenordnung von oder unterhalb von 0,23 Gew,% in einem intravaskulär injizierbaren Arzneimittelträger gelöst, um eine intravaskulär zu verabreichende Lösung zu erhalten, die bestimmt ist, Aids im ausgebrochenen oder sich entwickelnden Zustand zu bekämpfen.

13. Verwendung wenigstens eines Benzalkoniumfluorids der Formel 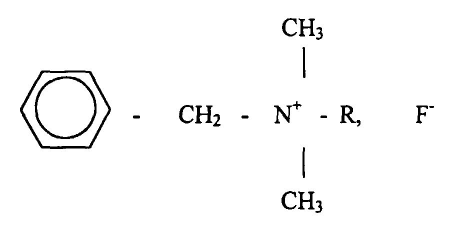 wobei R ein Alkylrest ist, der zwischen C₈H₁₇ und C₁₈H₃₇ variieren kann, zu einer Konzentration der Größenordnung von oder unterhalb von 0,23 Gew.% in einem intravaskulär injizierbaren Arzneimittelträger gelöst, um eine intravaskulär zu verabreichende Lösung zu erhalten, die bestimmt ist, Tuberkulose zu bekämpfen.

14. Verwendung nach einem der Ansprüche 11 bis 13, dadurch gekennzeichnet, daß Benzalkoniumfluorid in der Größenordnung von 0,2 Gew.% verwendet wird.

15. Verwendung nach einem der Ansprüche 11 bis 13, dadurch gekennzeichnet, daß mehr als 0,05 Gew.% Benzalkoniumfluorid verwendet wird.

16. Anwendung nach einem der Ansprüche 11 bis 15, dadurch gekennzeichnet, daß die Konzentration an Benzalkoniumfluorid bestimmt ist, um mit langsamer Injektion Dosen in der Größenordnung von 0,5 bis 23 mg - insbesondere der Größenordnung von 21 mg - Benzalkoniumfluorid pro Liter Blut des zu behandelnden Patienten verabreichen zu können.

17. Verwendung nach einem der Ansprüche 11 bis 14, dadurch gekennzeichnet, daß R von C₁₂H₂₅ und C₁₄H₂₉ umfaßt ist.

18. Verwendung nach einem der Ansprüche 12 bis 17, dadurch gekennzeichnet, daß zwischen 0,5 und 23 mg - insbesondere in der Größenordnung von 21 mg - Benzalkoniumfluorid, in 10 mL Lösung gelöst, verwendet wird.

19. Verwendung nach einem der Ansprüche 11 bis 18, dadurch gekennzeichnet, daß wenigstens ein Benzalkoniumfluorid in einer physiologischen Lösung gelöst ist.

20. Verwendung nach einem der Ansprüche 11 bis 19, dadurch gekennzeichnet, daß gleichzeitig wenigstens ein Metallderivat von Fluor verwendet wird.

21. Verwendung nach Anspruch 20, dadurch gekennzeichnet, daß gleichzeitig Lithiumfluorid verwendet wird.
